(19) Europäisches Patentamt / European Patent Office / Office européen des brevets

(11) EP 2 233 069 B1

## (12) FASCICULE DE BREVET EUROPEEN

(45) Date de publication et mention
de la délivrance du brevet:
**24.04.2013 Bulletin 2013/17**

(51) Int Cl.:
*A61B 5/0402* (2006.01)    *A61B 5/042* (2006.01)
*A61B 5/04* (2006.01)    *A61N 1/37* (2006.01)

(21) Numéro de dépôt: **10154061.5**

(22) Date de dépôt: **19.02.2010**

(54) **Dispositif médical actif comprenant des moyens de filtrage non-linéaire pour la reconstruction d'un électrocardiogramme de surface à partir d'un électrogramme endocavitaire**

Aktive medizinische Vorrichtung, die nicht-lineare Filtermittel zur Rekonstruktion eines Oberflächenelektrokardiogramms aus einer endokavitären Aktionsstromkurve umfasst

Active medical device including non-linear filtering means for reconstructing a surface electrocardiogram from an intracardiac electrogram

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **25.03.2009 FR 0901399**

(43) Date de publication de la demande:
**29.09.2010 Bulletin 2010/39**

(73) Titulaire: **Sorin CRM SAS**
**92541 Montrouge (FR)**

(72) Inventeur: **Makdissi, Alaa**
**75011, PARIS (FR)**

(74) Mandataire: **Dupuis-Latour, Dominique**
**Bardehle Pagenberg**
**10, boulevard Haussmann**
**75009 Paris (FR)**

(56) Documents cités:
**EP-A- 1 902 750    WO-A-2008/008692**

**Description**

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme.

L'invention concerne plus particulièrement une technique de traitement des signaux représentatifs des potentiels cardiaques de dépolarisation du myocarde, signaux recueillis par des électrodes épicardiques ou endocavitaires de stimulation ou de détection ou de défibrillation des oreillettes ou des ventricules droits et gauches de ces dispositifs implantés.

Plus précisément, l'invention propose une technique, mise en oeuvre ou non dans ce dispositif implanté, permettant de reconstruire un électrocardiogramme de surface (ECG) à partir d'un électrogramme endocavitaire ou épicardique (EGM).

Les signaux EGM sont des signaux recueillis par des électrodes placées sur les sondes endocavitaires ou épicardiques implantées avec le dispositif. Ces signaux sont directement issus de l'activité électrique des cellules du myocarde et fournissent une information très riche pour évaluer l'état du patient. Après amplification, numérisation et filtrage, ils sont utilisés pour piloter le stimulateur cardiaque et pour diagnostiquer certains troubles du rythme nécessitant le déclenchement automatique d'une thérapie antitachycardique, antibradycardique ou de resynchronisation interventriculaire.

En revanche, lorsqu'il s'agit d'analyser de façon subjective le rythme pour établir un diagnostic ou pour réajuster les paramètres d'un implant, les médecins préfèrent en pratique interpréter les informations d'un électrocardiogramme de surface (ECG), qui permettent de visualiser de façon directe un certain nombre de facteurs importants (largeur du QRS, ...) et apprécier l'évolution d'une insuffisance cardiaque.

Ce sont également les signaux ECG qui sont enregistrés sur une longue période en ambulatoire par les dispositifs Holter, pour être ensuite traités et analysés afin d'évaluer l'état clinique du patient et diagnostiquer le cas échéant un trouble du rythme cardiaque.

Or les signaux ECG et EGM, bien qu'ils aient la même source (l'activité électrique du myocarde), se présentent visuellement de façon assez différente : l'EGM recueilli par la prothèse cardiaque fournit une information locale sur l'activité électrique d'un groupe de cellules cardiaques, tandis que l'ECG apparaît sous une forme plus globale, influencée en particulier par la propagation du signal électrique entre le myocarde et la surface du corps, et par un certain nombre de facteurs morphologiques et pathologiques. De ce fait, pour un médecin habitué à interpréter de longue date des ECG de surface, les signaux EGM ne sont généralement pas parlants.

Par conséquent, lorsqu'un patient porteur d'un implant vient voir son médecin pour une visite de contrôle, ce dernier utilise en pratique deux appareils, à savoir un enregistreur ECG et un programmateur d'implant. Pour recueillir l'ECG, le praticien colle un certain nombre d'électrodes sur le torse du patient, de manière à définir les douze dérivations usuelles correspondant à autant de signaux ECG différents. Le programmateur est, quant à lui, utilisé pour contrôler certains paramètres de fonctionnement de l'implant (par exemple la durée de vie de la pile), télécharger des données enregistrées dans la mémoire de l'implant, modifier éventuellement le paramétrage de ce dernier, y télécharger une version modifiée du logiciel de commande, etc.

La consultation chez le médecin requiert donc généralement deux appareils différents, ainsi qu'une manipulation pour la pose des électrodes de surface et le recueil des signaux ECG.

Le recours à ces deux appareils implique de plus le déplacement du patient dans un centre spécialement équipé (disposant du programmateur spécifique adapté à l'implant qu'il porte), avec pour conséquence le plus souvent un espacement des visites de contrôle et donc un moindre suivi du patient.

Par ailleurs, l'enregistrement d'un ECG présente divers inconvénients, notamment :

- la préparation du patient qui nécessite un certain temps, avec pour corolaire une augmentation du coût global du suivi ;
- l'irritation locale que peut créer la fixation des électrodes sur la peau de certains patients ;
- la position des électrodes qui varie d'une visite à l'autre, induisant des variations dans l'ECG recueilli,
- le fait que l'ECG est affecté par plusieurs paramètres qu'il est difficile de maîtriser, comme la respiration, les mouvements du patient, ainsi que les interférences provenant de sources électriques externes.

Pour remédier à ces multiples inconvénients, on a cherché à développer des algorithmes de reconstruction de l'ECG de surface à partir des signaux EGM, c'est-à-dire à partir des signaux directement délivrés par la prothèse implantée.

Une reconstruction de l'ECG de surface à partir des signaux EGM permettrait en effet :

- d'éviter, lors de la consultation, d'avoir à poser des électrodes et à recourir à un enregistreur ECG, avec tous les inconvénients et les imprécisions liés à cette technique ;

- de rendre ainsi la consultation plus simple et plus rapide, éventuellement de pouvoir la faire à domicile et par voie de conséquence rapprocher les contrôles et améliorer le suivi du patient ;
- et d'autoriser éventuellement une télétransmission des données EGM enregistrées par la prothèse, sans intervention d'un praticien ou auxiliaire médical auprès du patient.

Divers algorithmes de reconstruction de l'ECG de surface à partir des signaux EGM ont ainsi été proposés.

Le US 5 740 811 A1 (Hedberg et al.) prévoit de synthétiser un signal ECG de surface par combinaison d'une pluralité de signaux EGM au moyen d'un réseau de neurones et/ou d'une logique floue et/ou d'un circuit sommateur, après apprentissage effectué par un algorithme du type *feedforward.*

L'un des inconvénients de cette technique est qu'elle n'introduit pas dans la chaîne de traitement de délai entre les signaux EGM et les signaux ECG, et ne permet donc pas de reconstruire de façon satisfaisante les signaux ECG. Un autre inconvénient tient au fait que cette technique ne tient pas compte de la position des sondes endocavitaires, qui peut changer entre le moment de l'apprentissage et celui de l'utilisation du dispositif : une modification de l'axe électrique du coeur aura ainsi pour effet de biaiser le signal ECG synthétisé, qui ne sera plus significatif, avec le risque de masquer un trouble du rythme qui ne sera pas diagnostiqué.

Le US 6 980 850 A1 (Kroll et al.) tente de remédier à cette difficulté par une méthode de reconstruction de l'ECG de surface mettant en oeuvre un filtrage linéaire, avec une transformation matricielle permettant de restituer individuellement chacune des dérivations de l'ECG de surface. La reconstruction consiste, par une matrice de transfert prédéterminée, à transformer un vecteur d'entrée représentatif d'une pluralité de signaux EGM en un vecteur résultant représentatif des différentes dérivations ECG. La matrice de transfert est apprise au cours d'une phase d'apprentissage par moyennage de plusieurs matrices instantanées à partir de vecteurs ECG et EGM enregistrés simultanément sur une même durée.

Cette technique, si elle constitue un progrès par rapport à celle du brevet précédent, présente cependant un certain nombre d'inconvénients. En premier lieu, elle suppose qu'il existe une relation linéaire entre les vecteurs ECG et EGM : or cette approximation, si elle se révèle satisfaisante avec des patients présentant un rythme régulier, entraîne en présence de morphologies de signal atypiques ou irrégulières des erreurs importantes de reconstruction de l'ECG dans un certain nombre de cas - correspondant précisément à des situations potentiellement pathologiques. De plus, en présence de bruit, la solution obtenue n'est pas unique, ce qui ne permet donc pas de reconstruire de façon satisfaisante les signaux ECG.

Les US 7 383 080 et US 2008/0065161 décrivent encore une autre technique, consistant à concaténer, d'une part, le *farfield* (signal lointain) ventriculaire observé sur l'électrode située dans l'oreillette avec, d'autre part, le signal *farfield* auriculaire observé sur l'électrode située dans le ventricule, pour reconstruire un signal ECG de surface. Pour raccorder les deux signaux lors de leur concaténation, le traitement prévoit de soustraire un décalage, puis de multiplier chaque signal par un facteur donné pour l'amplifier ou l'atténuer.

Dans le cas d'un patient présentant un rythme régulier, cette technique de concaténation est efficace, car les deux signaux *farfield* sont bien séparés. En revanche, pour un patient présentant un rythme irrégulier, donc potentiellement pathologique, les signaux *farfield* sont noyés dans les ondes P et R et ne peuvent pas être distingués de façon satisfaisante de ces dernières. En outre, le traitement très simple qui est proposé (application d'un gain et d'un décalage temporel) ne permet pas de reproduire la morphologie exacte des signaux ECG, qui ne sont reconstitués que sous forme d'une approximation très sommaire.

Le EP 1 902 750 A1 (ELA Medical) décrit une technique de reconstruction d'ECG utilisant une analyse en composantes principales (ACP) afin d'extraire un vectogramme endocardiaque (VGM) à partir duquel sera reconstruit un vectocardiogramme de surface (VCG) permettant à son tour, par une transformation inverse, d'obtenir les signaux reconstruits des diverses dérivations ECG. La reconstruction du VCG à partir du VGM s'effectue par apprentissage, notamment au moyen d'un réseau neuronal.

Ces diverses techniques présentent toutes un certain nombre d'inconvénients, tenant notamment au fait que les signaux EGM et ECG, s'ils ont la même origine, n'en présentent pas moins des caractéristiques très différentes.

En effet, l'activité électrique du coeur est le reflet des stimulations spontanées dues aux courants ioniques dans les cellules cardiaques, ou des stimulations artificielles produites par application d'un courant électrique à ces cellules. Les signaux EGM, recueillis directement par l'implant sur une ou plusieurs voies, sont le reflet des potentiels électriques présents au niveau du myocarde, tandis que les signaux ECG correspondent aux potentiels électriques enregistrés à la surface du corps, sur un certain nombre de dérivations, après propagation depuis le myocarde jusqu'à cette surface.

Une reconstruction satisfaisante des signaux ECG à partir des signaux EGM implique de prendre en compte la propagation des phénomènes électriques au travers du corps du patient, ainsi que la dépendance des potentiels transmembranaires des courants ioniques et des tenseurs de conductivité des tissus. Ces phénomènes ont été modélisés sous diverses formes, connues généralement sous le nom de "modèles bidomaine", qui peuvent être formulés sous forme d'équations différentielles non linéaires des potentiels électriques, contenant des termes linéaires, quadratiques et cubiques.

Or les techniques de reconstruction décrites dans les documents précités s'appuient toutes sur une relation linéaire simple entre signaux EGM et signaux ECG, sans tenir compte des connaissances physiologiques des modèles bidomaine, à la seule exception des techniques utilisant des réseaux neuronaux, qui introduisent des relations non linéaires entre les EGM et les ECG. Toutefois, la non-linéarité introduite par ces réseaux neuronaux consiste simplement en une fonction sigmoïde ou un limiteur, et elle est très différente de la non-linéarité physiologique du modèle bidomaine, qui se traduit par la présence d'un terme quadratique et d'un terme cubique.

Un autre inconvénient, propre à toutes ces techniques, est qu'elles ne permettent pas de vérifier que la reconstruction du signal ECG donne un résultat correct, encore moins de quantifier la qualité de cette reconstruction.

Or il serait souhaitable de pouvoir disposer d'un tel critère, notamment en fonction de l'utilisation que l'on souhaite faire des signaux ECG reconstruits : s'il s'agit de simplement déterminer la présence ou non d'un pic, d'un complexe, ..., il est possible de se contenter d'une qualité de reconstruction moyenne, tandis que si l'on souhaite un diagnostic précis, fondé sur des détails spécifiques de l'ECG ou des mesures effectuées sur celui-ci, alors une qualité de reconstruction supérieure sera nécessaire.

L'invention a pour but de remédier à ces divers inconvénients, en proposant une nouvelle technique de reconstruction de signaux ECG à partir de signaux EGM, avec un filtrage non-linéaire qui soit proche de la non-linéarité physiologique des modèles bidomaine, et qui permette également d'introduire un retard représentatif de la propagation des signaux électriques dans les tissus corporels.

Essentiellement, la technique de l'invention est basée sur une approche utilisant des filtres non-linéaires de Volterra pour représenter la relation entre signaux EGM et signaux ECG.

Les filtres de Volterra d'ordre 3 autorisent en effet l'introduction de termes quadratiques et cubiques et de retards, permettant ainsi de s'approcher de manière beaucoup plus réaliste des modèles bidomaire, c'est-à-dire des modèles représentatifs de la réalité de la propagation des phénomènes électriques dans le corps du patient entre le myocarde et la surface du corps.

L'invention a pour but de remédier à ces différents inconvénients en proposant un dispositif de traitement de signaux représentatifs de potentiels cardiaques de dépolarisation du myocarde, du type connu d'après le EP 1 902 750 A1 précité, qui correspond au préambule de la revendication 1, avec des moyens de reconstruction d'ECG comprenant : une pluralité d'entrées, recevant une pluralité correspondante de signaux EGM d'électrogramme endocavitaire ou épicardique acquis chacun sur une voie EGM respective ; au moins une sortie, délivrant un signal ECG d'électrocardiogramme de surface reconstruit, relatif à une dérivation ECG donnée ; et un filtre numérique non-linéaire avec un fonction de transfert propre à déterminer ledit signal ECG en fonction de ladite pluralité de signaux EGM.

De façon caractéristique de l'invention, le dispositif comprend les éléments énoncés dans la partie caractérisante de la revendication 1, en particulier le filtre numérique non-linéaire comprend un filtre de type Volterra dont la fonction de transfert comporte un terme linéaire et au moins un terme quadratique et/ou cubique.

La technique du filtrage de Volterra dans le domaine médical est déjà connue, notamment d'après le WO 2008/008692 A2, qui décrit un filtrage de Volterra appliqué aux signaux R-R représentatifs du rythme cardiaque. Mais ce rythme cardiaque est obtenu à partir des signaux ECG ou EGM par des procédés très éloignés de ceux de l'invention, sans reconstruction avec filtrage non-linéaire. En fait, le but recherché par ce document est la caractérisation de la variabilité du rythme cardiaque (HRV), problème spécifique qui n'a aucun rapport avec celui de l'invention. Il n'est en particulier jamais suggéré dans ce document d'utiliser le filtrage de Volterra pour générer un signal ECG d'électrocardiogramme de surface reconstitué à partir de signaux EGM directement délivrés par une prothèse implantée.

Le filtrage de Volterra est par ailleurs une technique très éloignée de celle mise en oeuvre par le EP 1 902 750 A1 précité car, bien que le traitement par réseau de neurones pour la reconstruction des VCG à partir des VGM y soit de type non-linéaire, il ne ressemble en aucun cas à un filtrage de type Volterra. En effet, un réseau de neurones applique une fonction non-linéaire seulement sur la valeur de l'entrée x[n] à un instant donné, tandis que dans un filtre de Volterra le filtrage linéaire est appliqué sur des termes quadratiques ou cubiques obtenus à partir des entrées x[n-k] considérées à plusieurs instants antérieurs différents.

Diverses formes de mises en oeuvre avantageuses de l'invention sont énoncées dans les sous-revendications.

Le dispositif de l'invention peut être implémenté sous diverses formes :

- dispositif implantable du type prothèse cardiaque de stimulation, resynchronisation, cardioversion et/ou défibrillation ;
- programmateur externe comprenant des moyens de téléchargement et d'analyse des signaux EGM recueillis et mémorisés par un implant ;
- moniteur de *home monitoring,* comprenant des moyens de téléchargement et d'analyse des signaux EGM recueillis et mémorisés par un implant, ainsi que des moyens de télétransmission automatique à un site distant des données ainsi recueillies ; et/ou

- serveur de données d'un site, recevant des données d'un moniteur distant de *home monitoring* comprenant des moyens de téléchargement des signaux EGM recueillis et mémorisés par un implant, ainsi que des moyens de télétransmission automatique au site des données ainsi recueillies.

On va maintenant décrire des exemples de mise en oeuvre de l'invention, en référence aux dessins annexés où les mêmes références numériques désignent d'une figure à l'autre des éléments identiques ou fonctionnellement semblables.

La Figure 1 est une représentation schématique de la technique de reconstruction de l'ECG, et d'apprentissage des coefficients des filtres.
La Figure 2 est un schéma par blocs illustrant une implémentation d'un filtre de Volterra d'ordre 2, pour un système à une entrée et une sortie.
La Figure 3 est un schéma par blocs d'un premier exemple de mise en oeuvre de l'invention, permettant la reconstruction d'une dérivation ECG à partir de trois voies EGM.
La Figure 4 est un schéma par blocs d'un deuxième exemple de mise en oeuvre de l'invention, permettant la reconstruction d'une dérivation ECG à partir de deux voies EGM, auriculaire et ventriculaire.
La Figure 5 est un schéma par blocs d'un troisième exemple de mise en oeuvre de l'invention, permettant la reconstruction d'une dérivation ECG au moyen d'un unique filtre de Volterra.

On va maintenant décrire plusieurs exemples de mise en oeuvre de l'invention. En ce qui concerne ses aspects logiciels, l'invention peut être réalisée par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.
L'invention peut notamment être appliquée aux dispositifs implantables tels que ceux des familles *Reply* et *Paradym* produits et commercialisés par ELA Médical, Montrouge, France. Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre de l'invention est à la portée de l'homme du métier, et elle ne sera pas décrite en détail.
L'invention peut toutefois être mise en oeuvre non seulement au sein d'un implant (traitement direct des signaux VGM), mais également dans un programmateur externe utilisé par un praticien par téléchargement et analyse des signaux cardiaques recueillis et mémorisés par un implant.
Dans une variante avantageuse, l'invention est également mise en oeuvre dans un moniteur de *home monitoring,* qui est un type particulier de programmateur dont le fonctionnement est entièrement automatisé et ne nécessite pas le recours à un praticien, et qui est notamment destiné à permettre la télétransmission à intervalles réguliers à un site distant des données recueillies et analysées, par exemple quotidiennement, de manière à assurer un suivi à distance du patient.
L'invention peut également être mise en oeuvre au niveau du serveur de données de ce site, les données EGM brutes étant télétransmises à ce serveur directement, sans traitement préalable. Le traitement est alors effectué par le serveur ou par un terminal (PC ou programmateur) connecté à ce dernier.

*Recueil des signaux EGM*

Il s'agit d'acquérir des signaux EGM sur une pluralité de "voies", correspondant chacune à une paire d'électrodes endocavitaires ou épicardiques reliées au boîtier de la prothèse cardiaque implantée.
Le choix des électrodes définissant ces voies dépend de la prothèse cardiaque considérée : stimulateur (pour le traitement des bradycardies), défibrillateur (pour le traitement des tachycardies et fibrillations) ou resynchroniseur (pour le traitement de l'insuffisance cardiaque). On distingue également trois modes de stimulation : simple, double ou triple chambre. À ces différentes fonctions correspondent des électrodes différentes, et un nombre de signaux EGM qui n'est pas le même selon le cas.
Si l'on désigne par "VD", "OD" et "VG", respectivement, les électrodes ventriculaire droite, auriculaire droite et ventriculaire gauche des sondes intracardiaques, avec un signe "+" ou "-" indiquant la position, distale ou proximale, de l'électrode, et par "CoilV" et "SVC" les électrodes de défibrillation respectivement ventriculaire et supraventriculaire, les combinaisons d'électrodes possibles sont les suivantes (avec, à chaque fois, possibilité de définir la voie entre deux électrodes ou bien entre l'une des électrodes et le boîtier du générateur) :

- simple chambre : VD+, VD- (et CoilV dans le cas d'un défibrillateur) ; un stimulateur simple chambre peut ainsi fournir deux signaux EGM grâce aux électrodes distale et proximale, la masse étant prise sur le boîtier. La version en défibrillateur pourra délivrer trois signaux EGM, grâce à l'électrode CoilV supplémentaire.

- double chambre : VD+, VD-, OD+, OD- (et CoilV et SVC dans le cas d'un défibrillateur) ; un stimulateur double chambre peut ainsi fournir quatre signaux EGM, et six dans la version défibrillateur.
- triple chambre : VD+, VD-, OD+, OD-, VG+, VG- (et CoilV et SVC dans le cas d'un défibrillateur) ; un stimulateur triple chambre peut ainsi fournir six signaux EGM, et huit dans la version défibrillateur.

*Principe de reconstruction de l'ECG*

Les signaux ECG, qui sont la manifestation de l'activité électrique cardiaque à la surface du corps du patient, sont normalement recueillis entre des paires d'électrodes appliquées en des endroits prédéterminés du thorax du patient, chaque paire d'électrodes déterminant une "dérivation". Le tout forme un ensemble de douze dérivations, comprenant des dérivations bipolaires (I, II, III), unipolaires (aVF, aVR, aVL) et précordiales (v1 à v6).

L'invention a pour objet de reconstituer les signaux d'une ou plusieurs de ces dérivations ECG à partir de signaux effectivement recueillis sur une pluralité de voies EGM.

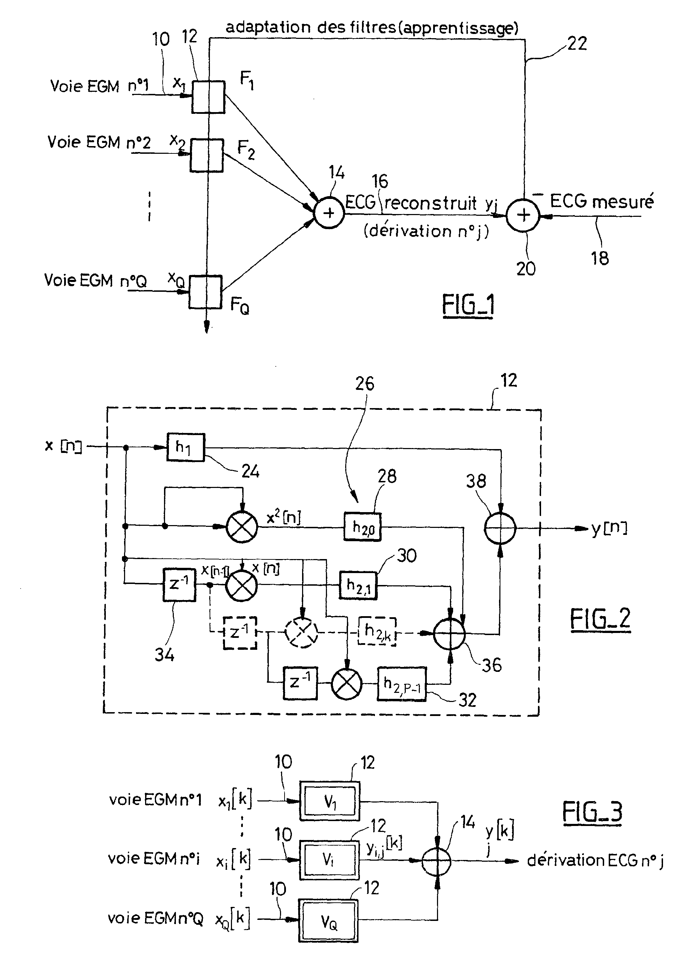

Le principe de base de cette reconstruction est schématisé sur la Figure 1.

Une pluralité de signaux EGM $x_1$, $x_2$ ... $x_Q$, sont recueillis et échantillonnés sur Q voies EGM 10, de la manière indiquée plus haut. Chacun de ces signaux est appliqué à un filtre respectif $F_1$, $F_2$, ... $F_Q$, référencé 12. Les signaux de sortie de chacun des ces filtres 12 sont combinés linéairement entre eux en 14 pour produire un signal ECG reconstruit 16, correspondant à l'une des douze dérivations que l'on souhaite reconstruire. Le signal reconstruit sur la dérivation n° $j$ sera désigné $y_j$. Pour obtenir les signaux des autres dérivations ECG que l'on souhaite également reconstruire, la même technique sera appliquée, avec des paramètres de filtre différents.

Le paramétrage des filtres correspondant à une dérivation ECG donnée est réalisé par apprentissage, en comparant le signal d'ECG reconstruit 16 à un signal ECG effectivement mesuré 18 sur la dérivation considérée. Ces deux signaux sont appliqués à un combineur linéaire différentiel 20 délivrant en 22 les données permettant d'assurer, par apprentissage, l'adaptation et le paramétrage des filtres 12. Cette étape d'apprentissage, qui sera exposée plus en détail dans la suite de la description, consiste essentiellement à calculer, pour chacun des filtres $F_1$, $F_2$, ... $F_Q$, les paramètres de ces filtres qui minimisent l'écart entre l'ECG reconstruit et l'ECG effectivement mesuré.

*Principe du filtrage de Volterra*

De façon caractéristique de l'invention, les filtres $F_1$, $F_2$, ... $F_Q$ sont des filtres non-linéaires de type filtre de Volterra.

Le filtrage de Volterra est par exemple décrit par Schetzen M, The Volterra and Wiener Theories of Nonlinear Systems, Wiley and Sons, New York, 1980, ou encore par Mathews VJ, Adaptive Polynomial Filters, IEEE Signal Processing Magazine, 8(3), pp.1 0-26, juillet 1991.

Ces filtres permettent en particulier d'établir entre les signaux EGM et les signaux ECG une relation non-linéaire similaire à celle des modèles bidomaine, c'est-à-dire incluant des termes linéaires, quadratiques et cubiques. Ils permettent également d'introduire dans cette relation un retard fini reflétant la propagation des signaux électriques dans les tissus corporels depuis le myocarde jusqu'à la surface de la peau du patient.

Plus précisément, un filtre de Volterra est un filtre qui, recevant en entrée un signal réel temporel discret $x[n]$, avec $n$ entier ($n$ étant le rang de l'échantillon du signal), génère la sortie $y[n]$ définie par :

$$y[n] = h_0 + \sum_{m=1}^{p} \sum_{k_1=0}^{N_1-1} \cdots \sum_{k_p=0}^{N_m-1} h_m[k_1, \cdots, k_m] \, x[n-k_1] \ldots x[n-k_m]$$

Cette équation est une relation entrée-sortie d'un système dénommé filtre de Volterra réel d'ordre $p$.

Dans la suite, pour simplifier l'exposé, l'ordre sera limité à $p = 3$, mais cette simplification n'est aucunement limitative, l'invention pouvant s'appliquer à des filtres d'ordre supérieur. Pour un ordre $p = 3$, l'équation précédente s'écrit alors :

$$y[n] = h_0 + \sum_{k=0}^{N-1} h_1[k]x[n-k] + \sum_{k=0}^{P-1}\sum_{l=0}^{P-1} h_2[k,l]x[n-k]x[n-l] + \sum_{k=0}^{M-1}\sum_{l=0}^{M-1}\sum_{r=0}^{M-1} h_3[k,l,r]x[n-k]x[n-l]x[n-r]$$

Cette équation peut être mise sous la forme simplifiée :

$$y[n] = h_0 + h_1(x[n], N) + h_2(x[n], P) + h_3(x[n], M)$$

où $h_0$ est un terme constant, indépendant de l'entrée, $h_1$ est un filtre linéaire, $h_2$ un filtre quadratique et $h_3$ un filtre cubique (le terme $h_m$ étant dénommé "noyau d'ordre m" du filtre),

Il est évident qu'un tel filtre est causal, puisqu'il ne fait intervenir que des valeurs antérieures à l'instant n.

N représente la "mémoire" du filtre linéaire $h_1$, correspondant à la part plus ou moins importante du passé de l'entrée intervenant pour la construction de la sortie présente, P et M représentant, de la même façon, la mémoire des filtres quadratique $h_2$ et cubique $h_3$. Dans la littérature, les filtres de Volterra ont généralement des mémoires N, M et P identiques mais, dans le cas de la présente invention, il est préférable d'utiliser des mémoires différentes pour chaque noyau $h_m$ afin de réduire la complexité global du calcul.

La figure 2 représente les détails d'une implémentation d'un filtre de Volterra. Pour simplifier l'exposé, on prendra l'exemple d'un filtre d'ordre 2, et sans terme constant.

Ce filtre 12 comprend une entrée x[n] et une sortie y[n]. Le noyau linéaire 24 (terme $h_1$) agit directement sur le signal d'entrée x[n]. Le noyau quadratique 26 (terme $h_2$), quant à lui, est composé de P filtres linéaires 28, 30 ... 32. Le premier filtre linéaire 28 de ce noyau $h_{2,0}$ agit sur le signal x[n] multiplié par lui-même, soit $x[n]x[n] = x^2[n]$. Le deuxième filtre linéaire 30 de ce noyau $h_{2,1}$ agit sur le signal x[n] multiplié par ce même signal retardé d'un échantillon (l'opérateur $z^{-1}$ du bloc 34 représentant un retard d'un échantillon). L'entrée de ce deuxième filtre 30 est alors le signal x[n].x[n-1]. Et ainsi de suite, le dernier filtre linéaire $h_{2,P-1}$ du noyau agissant sur le signal x[n].x[n-P-1].

Dans sa forme générale, le filtre quadratique $h_2$ est une matrice bidimensionnelle de PxP éléments, et le filtre cubique $h_3$ est une matrice tridimensionnelle de MxMxM éléments.

Si l'on élimine de ces matrices les termes redondants, on peut en réduire le nombre de coefficients, l'équation générale d'un filtre de Volterra de troisième ordre devenant alors :

$$y[n] = h_0 + \sum_{k=0}^{N-1} h_1[k]x[n-k]$$

$$+ \sum_{k \geq l}^{P-1}\sum_{l=0}^{P-1} h_2[k,l]x[n-k]x[n-l]$$

$$+ \sum_{k \geq l}^{M-1}\sum_{l \geq r}^{M-1}\sum_{r=0}^{M-1} h_3[k,l,r]x[n-k]x[n-l]x[n-r]$$

On remarquera que, si $h_0 = 0$, et si $P = 0$ et $M = 0$ (pas de mémoire du passé pour les noyaux quadratique et cubique), la relation précédente se réduit à la convolution classique définissant un filtre linéaire transverse, aussi dénommé à réponse impulsionnelle finie (FIR).

*Premier mode de mise en oeuvre de l'invention*

De façon générale, dans le cas de la reconstruction d'un ECG à partir des signaux EGM, le système possède plusieurs entrées (selon le nombre de voies EGM mesurables par l'implant) et une ou plusieurs sorties (de une à douze, selon le nombre de dérivations ECG souhaité). Un tel système est de type multi-entrée/multi-sortie (MIMO).

Pour simplifier l'exposé ci-dessous, chaque dérivation ECG sera considérée indépendamment, et l'on décrira la reconstruction d'une seule dérivation ECG à partir d'une pluralité de voies EGM d'entrée (système multi-entrée/sortie-unique MISO). La méthode de reconstruction pourra bien entendu être généralisée à une pluralité de dérivations ECG, chacune avec ses propres coefficients de filtrage.

Cette première mise en oeuvre est faite en référence à la Figure 3.

Un filtrage de Volterra (filtre 12) est appliqué à chacune des voies EGM 10. Les sorties des filtres 12 font ensuite l'objet d'une combinaison linéaire (additionneur 14), pour donner la dérivation ECG reconstruite à partir de l'ensemble des voies filtrées.

Si l'on désigne $x_i[k]$ le signal discret de la voie EGM n° $i$, et $h_m^{i,j}$ le noyau d'ordre $m$ du filtre de Volterra attaqué par ce signal $x_i[k]$ pour reconstruire le signal $y_j[k]$ représentant la dérivation ECG (reconstruite) n° $j$, la sortie $y_{i,j}[k]$ du filtre de Volterra 12 correspondant est donnée par :

$$y_{i,j}[n] = h_0^{i,j} + \sum_{k=0}^{N-1} h_1^{i,j}[k]x_i[n-k]$$

$$+ \sum_{k\geq l}^{P-1}\sum_{l=0}^{P-1} h_2^{i,j}[k,l]x_i[n-k]x_i[n-l]$$

$$+ \sum_{k\geq l}^{M-1}\sum_{l\geq r}^{M-1}\sum_{r=0}^{M-1} h_3^{i,j}[k,l,r]x_i[n-k]x_i[n-l]x_i[n-r]$$

Le signal $y_j[k]$ de la dérivation ECG n° $j$ reconstruit à partir des Q signaux $y_{i,j}[k]$ est donné par :

$$y_j[n] = \sum_{i=1}^{Q} y_{i,j}[n]$$

On démontre que le nombre d'inconnues à déterminer pour reconstruire une (unique) dérivation ECG à partir de Q voies EGM est égal à :

$$K_1 = 1 + Q[N + P(P+1)/2 + M(M+1)(M+2)/6].$$

Il est possible d'annuler certains noyaux $h_m$ pour réduire le nombre d'inconnues dans le système à résoudre, par exemple en n'utilisant que des filtres de Volterra quadratiques d'ordre 2, donc avec $M = 0$. On peut aussi choisir d'annuler le terme quadratique ($P = 0$), en gardant le terme cubique.

*Deuxième mode de mise en oeuvre de l'invention*

Cette deuxième mise en oeuvre du procédé utilise une version simplifiée du filtre de Volterra, qui ne prend en compte que les produits $x_i[n-k]x_i[n-k]$, c'est-à-dire entre des échantillons temporels du même signal d'entrée $x_i[k]$ pris aux mêmes instants d'échantillonnage. Ceci revient à appliquer des filtres linéaires aux signaux $x_i[k]$, à leurs carrés $x_i[k]x_i[k] = x_i^2[k]$ ,... etc. (termes diagonaux des noyaux $h_m$). La contribution du signal $x_i[k]$ de la voie EGM n° i dans la construction de la dérivation ECG $y[k]$ est alors :

$$y_i'[n] = h_0^i + \sum_{k=0}^{N-1} h_1^i[k]x_i[n-k] + \sum_{k=0}^{P-1} h_2^i[k]x_i^2[n-k] + \sum_{k=0}^{M-1} h_3^i[k]x_i^3[n-k]$$

Avec cette version simplifiée, il peut être avantageux de prendre également en compte les produits croisés $x_c[k]$ entre plusieurs entrées EGM aux mêmes instants d'échantillonnage $x_c[k] = x_1[k]x_2[k]\cdots x_Q[k]$. L'équation du signal ECG reconstruit s'écrit alors :

$$y\ [n] = \sum_{i=1}^{Q} y_i'\ [n] + \sum_{s=0}^{N-1} h_c[s]x_c[n-k]$$

Le nombre d'inconnues est : 1 pour le terme constant, $QN$ pour le terme linéaire, $QP$ pour le terme quadratique, $QM$ pour le terme cubique et $N$ pour le terme des produits croisées, soit en tout $K_2 = 1 + N + Q(N + P + M)$ inconnues à déterminer.

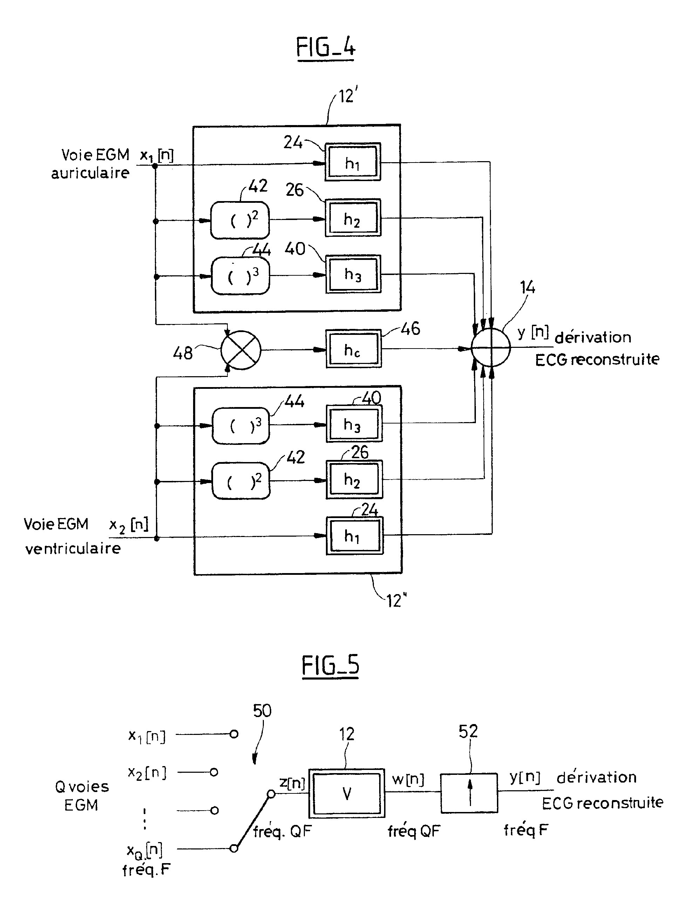

La figure 4 est un schéma par blocs illustrant cette deuxième mise en oeuvre, dans le cas particulier de deux voies EGM en entrée, à savoir :

- le signal $x_1[n]$ qui représente l'EGM recueilli par une sonde situé dans l'oreillette, et
- le signal $x_2[n]$ qui représente l'EGM recueilli par une sonde situé dans le ventricule.

Le bloc 12' représente un premier filtre de Volterra simplifié, agissant sur les termes diagonaux seulement. Il contient trois filtres linéaires 24, 26, 40 représentant les trois noyaux de ce filtre, qui sont respectivement attaqués par le signal $x_1[n]$, son carré 42 et son cube 44. Le bloc 12" effectue les mêmes opérations sur le signal $x_2[n]$.

Le filtre $h_c$ 46, quant à lui, est un filtre linéaire attaqué par le produit croisé 48 des signaux $x_1[n]$ et $x_2[n]$. Dans le cas où $x_1[n]$ est le signal recueilli dans l'oreillette et $x_2[n]$ le signal recueilli dans le ventricule, le produit croisé $x_c[k] = x_1[k]x_2[k]$ représente un signal proche du signal *far-field* car le signal $x_2[n]$ est atténué partout sauf aux moments de l'onde P, et le signal $x_1[n]$ est atténué partout sauf aux moments de l'onde R. En d'autres termes, le signal $x_c[k]$ représente le signal de l'onde P, amplifié aux moments de l'onde R.

Le signal ECG est calculé par une combinaison linéaire 14 des sorties des filtre de Volterra 12' et 12" et de la sortie du filtre $h_c$ 46 (le terme constant $h_0$ étant omis, sur cette figure).

Cette version simplifiée présente l'avantage d'une complexité réduite par rapport à la version complète de la première mise en oeuvre, tout en tenant compte des non-linéarités du modèle bidomaine.

Comme dans le cas de la première mise en oeuvre, il est possible de réduire davantage la complexité du filtre de Volterra de la deuxième mise en oeuvre en annulant certains noyaux, d'ordre 2 ou 3 notamment.

*Troisième mode de mise en oeuvre de l'invention*

Cette troisième mise en oeuvre de l'invention n'utilise qu'un seul filtre de Volterra pour la reconstruction d'une dérivation ECG, quel que soit le nombre $Q$ de voies EGM utilisées à cet effet.

La technique utilisée, illustrée en référence à la Figure 5, consiste à produire, un signal $z[n]$ constitué d'une concaténation de signaux EGM successifs $x_i[n] = x_1[n]$, $x_2[n]$ ... $x_Q[n]$. Pour chaque instant d'échantillonnage nT, le signal z est constitué en utilisant l'ensemble des Q valeurs des signaux $x_i[n]$. De ce fait, si la fréquence d'échantillonnage des signaux EGM est $F$, celle du signal $z[n]$ sera égale à $QF$.

La fonction 50 peut être mise en oeuvre par un convertisseur parallèle/série, ou par un interrupteur circulaire 50 basculant entre les Q signaux d'entrée à une fréquence $QF$.

Un filtrage de Volterra (filtre 12) est ensuite appliqué au signal $z[n]$ ainsi formé, le filtrage produisant en sortie un signal $w[n]$, également à fréquence $QF$.

Le signal $y[n]$, à fréquence $F$, est extrait du signal $w[n]$ par décimation (sous-échantillonnage) d'ordre $Q$, c'est-à-dire en gardant une valeur tous les $Q$ valeurs (bloc 52).

Cette mise en oeuvre fait intervenir des produits croisés entre les valeurs de différents signaux d'entrée, prises aux différents instants d'échantillonnage. Puisque le filtre de Volterra fonctionne à fréquence $QF$, pour couvrir le même passé du signal il nécessitera $Q$ fois plus de longueur de mémoire par noyau qu'un même filtre fonctionnant à fréquence $F$. Le nombre d'inconnues à déterminer pour reconstruire une voie ECG à partir de $Q$ voies EGM sera :

$$K_3 = 1 + [QN + QP(QP+1)/2 + QM(QM+1)(QM+2)/6]$$

La complexité du filtre de Volterra sera essentiellement fonction du nombre $Q$ de voies EGM appliquées en entrée.

*Détermination des coefficients des filtres de Volterra*

Comme on l'a exposé plus haut, la détermination des coefficients des filtres de Volterra utilisés pour reconstruire l'ECG repose sur un apprentissage opéré, dans une première phase, par recueil simultané d'un jeu de données de référence constitué de signaux EGM $x_i^R[k]$ et de signaux ECG $y^R[k]$ synchronisés avec ces signaux EGM.

Le nombre d'inconnues à déterminer dans le système dépend des tailles des mémoires N, P et M des filtres, et la longueur de la séquence d'apprentissage doit être suffisante pour permettre une détermination non ambiguë des coefficients du filtre (supérieure au nombre d'inconnues dans le système de Volterra).

Les équations de filtrage développées ci-dessus vont être exprimées sous une forme plus simple. L'idée de base étant que la sortie *y* est globalement linéaire par rapport à l'ensemble des paramètres *h*, bien qu'elle soit non linéaire par rapport l'entrée *x*, ceci permet d'écrire la relation entrée-sortie sous la forme d'un produit scalaire de vecteurs convenablement définis.

Dans le cas du premier mode de mise en oeuvre de l'invention, on forme un vecteur d'inconnues **H,** de taille égale à $K_1$ défini ci-dessus, par concaténation de plusieurs sous-vecteurs $\mathbf{h}_m^i$ représentant chacun les coefficients du noyau d'ordre *m* attaqué par le signal de la voie EGM n° *i* :

$$\mathbf{H} = \left[\mathbf{h}_0, \mathbf{h}_1^1, \mathbf{h}_2^1, \mathbf{h}_3^1, \cdots, \mathbf{h}_1^Q, \mathbf{h}_2^Q, \mathbf{h}_3^Q\right]^T$$

Cette simplification des noyaux peut être reprise pour le signal d'entrée. En particulier, on peut associer au vecteur $\mathbf{x}_i[n] = \{x_i[n],...,x_i[n\text{-}N\text{-}1]\}$ de l'entrée EGM n° *i*, le vecteur $\mathbf{x}_m^i$ représentant les termes non redondants qui interviennent dans le noyau d'ordre *m* :

$$\mathbf{x}_1^i[n] = \mathbf{x}_i[n]$$

$$\mathbf{x}_2^i[n] = \left\{x_i[n-k]x_i[n-l]\right\}_{P>k\geq l\geq 0}$$

$$\mathbf{x}_3^i[n] = \left\{x_i[n-k]x_i[n-l]x_i[n-r]\right\}_{M>r\geq k\geq l\geq 0}$$

et un vecteur global **X** représentant la contribution de l'ensemble des *Q* entrées EGM :

$$\mathbf{X}[n] = \left[1, \mathbf{x}_1^1[n], \mathbf{x}_2^1[n], \mathbf{x}_3^1[n], \cdots, \mathbf{x}_1^Q[n], \mathbf{x}_2^Q[n], \mathbf{x}_3^Q[n]\right]^T$$

(la constante 1 représentant le signal d'entrée pour le terme constant $h_0$ du filtre).

On constate alors que la relation entrée-sortie exposée plus haut pour le premier mode de mise en oeuvre peut prend la forme simplifiée :

$$y[n] = \mathbf{X}[n]^T \mathbf{H}$$

Dans le cas des deuxième et du troisième modes de mise en oeuvre, il devient alors possible de transformer les équations en un produit scalaire de deux vecteurs, à savoir un vecteur **H** contenant les coefficients du filtre et un vecteur **X** contenant des quantités calculées à partir des produits formés entre les valeurs des entrées aux différents instants d'échantillonnage.

*Détermination des coefficients du filtre (première technique - régularisation de Tikhonov)*

Disposant d'un jeu de référence contenant des signaux EGM et des signaux ECG acquis simultanément, l'équation précédente permet de déduire le filtre **H** qui vérifie au mieux cette équation.

Plusieurs techniques peuvent être mise en oeuvre pour aboutir à ce résultat.

Une première technique, matricielle, consiste à transformer l'équation vectorielle précédente en une équation matricielle, en utilisant l'ensemble des données.

En supposant que l'ensemble de données a été déjà acquis auparavant, la transformation peut être effectuée en différé (*batch mode*).

Soit $L$ la taille de jeu de données de référence, et $n_0$ = max($N, P, M$) la mémoire maximale des noyaux du filtre de Volterra. On dispose alors de $L\text{-}n_0$ équations vectorielles qu'il est possible de regrouper sous la forme :

$$\begin{bmatrix} y^R[n_0] \\ \vdots \\ y^R[n] \\ \vdots \\ y^R[L-n_0] \end{bmatrix} = \begin{bmatrix} \mathbf{X}_R[n_0]^T \\ \vdots \\ \mathbf{X}_R[n]^T \\ \vdots \\ \mathbf{X}_R[L-n_0]^T \end{bmatrix} \mathbf{H}$$

ou encore

$$\mathbf{Y} = \overline{\mathbf{A}}\, \mathbf{H}$$

**Y** étant un vecteur de $L\text{-}n_0$ valeurs d'ECG,

$\overline{\mathbf{A}}$ étant une matrice de $(L\text{-}n_0) \times K_1$ éléments non redondants formés à partir des entrées EGM, et

**H** étant un vecteur de $K_1$ inconnues représentant les coefficients du filtre de Volterra.

Le fait que la matrice $\overline{\mathbf{A}}$ soit composée d'éléments formellement non redondants ne garantit pas qu'elle soit bien conditionnée numériquement pour garantir une solution **H** unique et numériquement stable obtenue en résolvant le système linéaire (en **H**) ci-dessus. On notera également que les valeurs d'ECG dans **Y** et les valeurs d'EGM utilisées dans $\overline{\mathbf{A}}$ sont entachées de différents types de bruit. On parle alors d'un "problème inverse mal-posé" ou d'une "matrice mal-conditionnée".

La solution des moindres carrés du système linéaire précédent qui minimise la norme de l'écart entre les valeurs d'ECG recueillies **Y** et les valeurs d'ECG calculées par la solution $\mathbf{H}_{MC}$ est donnée par :

$$\mathbf{H}_{MC} = \left(\overline{\mathbf{A}}^{\mathrm{T}}\overline{\mathbf{A}}\right)^{-1}\overline{\mathbf{A}}^{\mathrm{T}}\mathbf{Y}$$

Cette solution $\mathbf{H}_{MC}$ minimise le critère des moindres carrés $\|\mathbf{Y}\text{-}\overline{\mathbf{A}}\,\mathbf{H}\|^2$. Afin de garantir une solution numériquement stable, l'invention propose d'utiliser une régularisation consistant à trouver une solution vérifiant un critère composite, en ajoutant une contrainte à la contrainte des moindres carrés. Cette régularisation, appelée "régularisation de Tikhonov" est exposée, dans son principe général, notamment par Tikhonov AN et Arsenin VA, *Solution of Ill-posed Problems,* Winston & Sons, Washington, 1977 (ISBN 0-470-99124-0). Le critère composite utilisé s'écrit :

$$\left\|\mathbf{Y} - \overline{\mathbf{A}}\,\mathbf{H}\right\|^2 + \lambda\left\|\overline{\Gamma}\,\mathbf{H}\right\|^2$$

$\overline{\Gamma}$ est la matrice de Tikhonov. Souvent cette matrice est choisie comme la matrice Identité $\overline{\Gamma} = \mathbf{I}$, pour favoriser

les solutions avec une norme faible pour garantir une stabilité numérique. Dans d'autres cas, la matrice $\overline{\Gamma}$ peut présenter un opérateur de différence du premier ordre pour favoriser les solutions lisses. Le paramètre $\lambda$ est appelé paramètre de régularisation et représente un compromis entre le critère des moindres carrés et le critère supplémentaire. La solution régularisée est donnée par :

$$\mathbf{H}_{Tikh} = \left(\overline{\mathbf{A}}^{\mathrm{T}}\overline{\mathbf{A}} + \lambda\,\overline{\mathbf{\Gamma}}^{\mathrm{T}}\overline{\mathbf{\Gamma}}\right)^{-1}\overline{\mathbf{A}}^{\mathrm{T}}\mathbf{Y}$$

On trouve dans la littérature plusieurs techniques pour le choix du paramètre de régularisation $\lambda$, la méthode de "validation croisée" étant la plus répandue.

Cette première technique de détermination des coefficients du filtre de Volterra permet d'obtenir une excellente qualité de reconstruction.

En revanche, elle n'est pas utilisable en temps réel, car elle nécessite le regroupement des données de référence dans une matrice. Elle exige aussi une grande taille de mémoire, car la taille de la matrice formée dépend de la taille des données de référence. Elle est préférentiellement implémentée dans un programmateur communiquant avec l'implant et disposant d'un processeur rapide et d'une grande taille de mémoire.

*Détermination des coefficients du filtre (deuxième technique - moindres carrés récursifs)*

Une autre technique utilisable pour déterminer les coefficients du filtre est la méthode des moindres carrés récursive (RLS, Recursive Least Squares), décrite par exemple par Hayes, MH (1996), Recursive Least Squares, Statistical Digital Signal Processing and Modeling, Wiley, p. 541 (ISBN 0-471-59431-8), ou encore par Haykin S, Adaptive Filter Theory, Prentice Hall, 2002 (ISBN 0-13-048434-2).

Cette méthode permet de résoudre en temps réel le système linéaire $y[n]=\mathbf{X}[n]^{T}\,\mathbf{H}$, et demande mois de ressources informatiques pour le calcul des coefficients. Elle peut donc être implémentée dans un implant sans le besoin d'un programmateur externe.

Cette méthode itérative utilise un pas variable pour contrôler la convergence lors des itérations, ce qui représente un avantage dans les systèmes multi-entrées par rapport aux méthodes itératives à pas fixe comme le LMS. Son facteur d'oubli joue le rôle de la régularisation de la méthode matricielle de Tikhonov.

*Évaluation de la qualité de la reconstruction de l'ECG*

Un autre aspect de la présente invention réside dans l'évaluation de la qualité de la reconstruction de l'ECG.

Il est en effet intéressant de pouvoir estimer la qualité de cette reconstruction, par exemple pour choisir telle ou telle technique de reconstruction sur la base d'un compromis acceptable entre les contraintes matérielles (temps de calcul, ressources matérielles et logicielles disponibles, ...) et les besoins réels selon l'utilisation que l'on souhaite faire de l'ECG reconstruit (détection de la simple présence de certaines caractéristiques, ou au contraire examen plus poussé des formes d'onde).

Pour évaluer cette qualité, les signaux EGM et ECG (signaux ECG réellement recueillis) sont acquis simultanément, pendant une durée $T_n$ de mesure. Cette durée $T_m$ doit représenter au moins deux cycles cardiaques (2 s environ) et peut aller jusqu'à 100 ou 1000 s. Les séquences choisies comme jeu de données de référence pour l'apprentissage ont une durée $T_r$ d'une seconde au moins et peuvent aller jusqu'à 99 ou 999 s. Les signaux EGM et ECG sont acquis simultanément pendant une durée $T_m$, typiquement avec un rythme d'échantillonnage de 128 Hz.

Si les fréquences d'échantillonnage des ECG et des EGM (en général situées dans la plage 100 Hz à 1 kHz) sont différentes, il faudra synchroniser les données par une technique appropriée, telle qu'une interpolation (linéaire, polynomiale ou par des *splines*) ou une compression (par exemple par l'algorithme de *turning point* de Mueller).

La qualité de la reconstruction des signaux ECG sera évaluée par un critère numérique consistant à déterminer, sur une séquence qui n'a pas été utilisée pour l'apprentissage, le coefficient de corrélation $\rho$ entre les signaux ECG réels $y[k]$ et les signaux ECG reconstruits $y_{rec}[k]$.

Concrètement, une erreur de séquencement de l'ordre de 40 ms (soit un décalage de $d$ = 5 échantillons pour une fréquence d'échantillonnage de 128 Hz) dans les signaux reconstruits n'altère pas les capacités de diagnostic des signaux ECG.

Le coefficient de corrélation est évalué pour chaque décalage $k$ :

$$\rho_k = \frac{\sum_{i=0}^{J} (y[i] - \bar{y})(y_{rec}[i+k] - \bar{y}_{rec})}{\sqrt{\sum_{i=0}^{J} (y[i] - \bar{y})^2} \sqrt{\sum_{i=0}^{J} (y_{rec}[i+k] - \bar{y}_{rec})^2}}$$

La qualité de reconstruction (comprise entre -1 et +1) est estimée par :

$$\rho = \max_{-d \le k \le d} (\rho_k)$$

Sur des séquences ECG qui présentent un rythme régulier, le procédé de l'invention assure une qualité de reconstruction supérieure à 97% à partir de signaux bipolaires provenant de l'oreillette et du ventricule (le signal proximal étant considéré par rapport au signal distal) pour un patient présentant un rythme régulier. Les signaux ECG reconstruit reproduisent fidèlement la polarité, la largeur et la position des complexes QRS dans les ECG.

Cette qualité de reconstruction très élevée est à comparer à celle obtenue avec les méthodes à base de réseaux neuronaux connues ayant le même nombre d'inconnues que le filtre de Volterra, qui est de l'ordre de 85 % seulement, en utilisant les signaux bipolaires provenant de l'oreillette et du ventricule chez un patient présentant un rythme cardiaque régulier.

Concrètement, la qualité de reconstruction doit être de l'ordre d'au moins 60 à 65 % pour pouvoir retrouver dans l'ECG reconstruit certaines singularités dont souhaite déterminer la présence ou l'absence (pics, complexes, ...), ce qui est suffisant pour un simple suivi de type monitoring ECG. En revanche, pour pouvoir établir un diagnostic plus précis à partir de l'examen détaillé des formes d'ondes, la qualité de reconstruction doit être d'au moins 80 %.

Le critère de qualité de reconstruction peut en particulier être utilisé pour valider le calcul des coefficients d'un filtre de la procédure d'apprentissage.

Ainsi, à l'issue du calcul des paramètres du filtre, la qualité de reconstruction est comparée à un seuil donné. Ce seuil est programmable et peut être éventuellement modifié par le praticien, ou peut être prédéfini à une valeur acceptable, par exemple 60 %.

Si le critère de qualité est vérifié (seuil dépassé), les coefficients calculés sont sauvegardés, et le filtre calculé sera utilisé pour la reconstruction ultérieure de la dérivation ECG considérée. Le processus est éventuellement répété pour une autre dérivation ECG.

En revanche, si le critère n'est pas vérifié, il y a lieu de recommencer la détermination des paramètres du filtre, soit en sélectionnant une autre période de référence $T_r$ dans la fenêtre de mesure $T_m$ (dans la fenêtre $T_r$, il y avait peut-être des arythmies susceptibles de perturber l'apprentissage), ou bien en recommençant l'acquisition d'un autre jeu de données sur une autre durée $T_m$.

**Revendications**

1. Un dispositif de traitement de signaux représentatifs de potentiels cardiaques de dépolarisation du myocarde, comportant des moyens de reconstruction d'ECG avec :

   - une pluralité d'entrées, recevant une pluralité correspondante de signaux EGM d'électrogramme endocavitaire ou épicardique ($x_1$, $x_2$ ... $x_Q$) acquis chacun sur une voie EGM respective (10) ;
   - au moins une sortie, délivrant un signal ECG d'électrocardiogramme de surface reconstruit. ($y_j$), relatif à une dérivation ECG donnée (16) ; et
   - un filtre numérique non-linéaire (12, 14 ; 12', 12", 14 ; 12, 52) avec un fonction de transfert propre à déterminer ledit signal ECG en fonction de ladite pluralité de signaux EGM,

   **caractérisé en ce que**
   ledit filtre numérique non-linéaire comprend un filtre de type Volterra ($Fi$, $F_2$ ... $F_Q$) dont la fonction de transfert comporte un terme linéaire ($h_1$), au moins un terme quadratique ($h_2$) et/ou cubique ($h_3$), et au moins un retard temporel fini ($z^{-1}$),
   ce filtre de Volterra i) recevant en entrée le signal EGM ($x_i[k]$) de l'une desdites voies EGM respectives et ii) générant en sortie un signal ECG correspondant filtré ($y_{i,j}[k]$).

2. Le dispositif de la revendication 1, comprenant :

- une pluralité de filtres non-linéaires (12) de Volterra recevant chacun en entrée le signal EGM ($x_i[k]$) de l'une desdites voies EGM respectives (10), et délivrant en sortie un signal correspondant filtré ($y_{i,j}[k]$); et
- des moyens (14) de combinaison linéaire des signaux filtrés par cette pluralité de filtres non-linéaires, délivrant en sortie ledit signal ECG reconstruit ($y_j[k]$).

3. Le dispositif de la revendication 1, comprenant :

- pour chacune des voies EGM : un premier filtre linéaire (24) recevant en entrée le signal EGM correspondant ($x_1 [n]$, $x_2, [n]$), un deuxième filtre linéaire (26) recevant en entrée le même signal EGM élevé au carré (42), et/ou un troisième filtre linéaire (40) recevant en entrée le même signal EGM élevé au cube (44) ; et
- des moyens (14) de combinaison linéaire des signaux filtrés par les premier, deuxième et/ou troisième filtres linéaires de chacune des voies EGM, délivrant en sortie ledit signal ECG reconstruit ($y[n]$).

4. Le dispositif de la revendication 3, comprenant en outre :

- un filtre linéaire additionnel (46) recevant en entrée une produit croisé (48) des signaux de chacune des voies EGM ;
les moyens de combinaison linéaire combinant en outre aux signaux filtrés par les premier, deuxième et/ou troisième filtres linéaires de chacune des voies EGM le signal délivré par ce filtre additionnel.

5. Le dispositif de la revendication 4, dans lequel les voies EGM sont au nombre de deux, avec une voie EGM auriculaire et une voie EGM ventriculaire.

6. Le dispositif de la revendication 1, comprenant :

- des moyens (50) pour concaténer des séquences d'échantillons ($x_1[n]$, $x_2[n]$ ... $x_Q[n]$) produits à fréquence F sur chacune des Q voies EGM respectives, et délivrer en sortie un signal concaténé ($z[n]$) de fréquence QF ;
- un filtre non-linéaire de Volterra (12) recevant en entrée ledit signal concaténé ($z[n]$) et délivrant en sortie un signal correspondant filtré ($w[n]$) de fréquence QF ; et
- des moyens de sous-échantillonnage 1/Q dudit signal filtré ($w[n]$), délivrant en sortie à une fréquence F ledit signal ECG reconstruit (y[$n$]).

7. Le dispositif de la revendication 1, comportant en outre des moyens de détermination préalable des paramètres du filtre numérique, comprenant :

- des moyens de recueil simultané de signaux EGM acquis (10) et d'au moins un signal ECG acquis (18) ; et
- des moyens (20, 22) de détermination des paramètres du filtre numérique, par minimisation d'un écart entre ledit signal ECG acquis (18) et un signal ECG reconstruit (16) à partir desdits signaux EGM (10) au moyen dudit filtre numérique (12, 14).

8. Le dispositif de la revendication 7, dans lequel les moyens (20, 22) de détermination des paramètres du filtre numérique sont des moyens de calcul direct desdits paramètres, aptes à mettre en oeuvre un algorithme de calcul matriciel donnant une solution vérifiant un critère de minimisation des moindres carrés.

9. Le dispositif de la revendication 8, dans lequel les moyens (20, 22) de détermination des paramètres du filtre numérique sont des moyens aptes à mettre en oeuvre un algorithme de calcul matriciel donnant une solution vérifiant un critère composite combinant la minimisation des moindres carrés à une contrainte de type régularisation de Tikhonov.

10. Le dispositif de la revendication 7, dans lequel les moyens (20, 22) de détermination des paramètres du filtre numérique sont des moyens de calcul itératif desdits paramètres, aptes à mettre en oeuvre un algorithme de type moindres carrés récursif RLS (*Recursive Least Squares*) à pas variable.

11. Le dispositif de la revendication 1, comportant en outre des moyens d'évaluation de la qualité de la reconstruction opérée par le filtre numérique non-linéaire, comprenant :

- des moyens de recueil simultané de signaux EGM acquis et d'au moins un signal ECG acquis ;
- des moyens de détermination d'un signal ECG reconstruit à partir desdits signaux EGM acquis ; et
- des moyens de calcul d'un coefficient de corrélation entre le signal ECG acquis et le signal ECG reconstruit.

**12.** Le dispositif de la revendication 11, comprenant en outre :

- des moyens de détermination préalable des paramètres du filtre numérique, et
- des moyens de validation des paramètres qui ont été ainsi déterminés, ces moyens comprenant des moyens pour comparer à un seuil donné le coefficient de corrélation calculé par lesdits moyens d'évaluation de la qualité de la reconstruction, et pour valider ou infirmer lesdits paramètres en fonction du résultat de cette comparaison.

**13.** Le dispositif de la revendication 1, dans lequel ladite pluralité d'entrées sont des entrées aptes à recevoir une pluralité correspondante de signaux EGM acquis à partir d'électrodes choisies parmi : électrode ventriculaire droite distale et/ou proximale, électrode auriculaire droite distale et/ou proximale, électrode ventriculaire gauche distale et/ou proximale, bobine de défibrillation ventriculaire ou auriculaire, bobine de défibrillation supra-ventriculaire.

**14.** Le dispositif de la revendication 1, où ledit dispositif est un dispositif implantable du type prothèse cardiaque de stimulation, resynchronisation, cardioversion et/ou défibrillation.

**15.** Le dispositif de la revendication 1, où ledit dispositif est un programmateur externe comprenant des moyens de téléchargement et d'analyse des signaux EGM recueillis et mémorisés par un implant.

**16.** Le dispositif de la revendication 1, où ledit dispositif est un moniteur de *home monitoring,* comprenant des moyens de téléchargement et d'analyse des signaux EGM recueillis et mémorisés par un implant, ainsi que des moyens de télétransmission automatique à un site distant des données ainsi recueillies.

**17.** Le dispositif de la revendication 1, où ledit dispositif est un serveur de données d'un site, recevant des données d'un moniteur distant de *home monitoring* comprenant des moyens de téléchargement des signaux EGM recueillis et mémorisés par un implant, ainsi que des moyens de télétransmission automatique audit site des données ainsi recueillies.

**Claims**

**1.** A device for processing signals representative of myocardial depolarisation cardiac potentials, comprising ECG reconstruction means with:

- a plurality of inputs, receiving a corresponding plurality of endocavitary or epicardial electrogram EGM signals ($x_1$, $x_2$ ... $x_Q$), each acquired on a respective EGM channel (10);
- at least one output, delivering a reconstructed surface electrocardiogram ECG signal ($y_j$), relating to a given ECG derivation (16); and
- a non-linear digital filter (12, 14; 12', 12", 14; 12, 52) with a transfer function adapted to determine said ECG signal as a function of said plurality of EGM signals,
**characterized in that** said non-linear digital filter comprises a filter of the Volterra type ($F_{i1}$, $F_2$ ... $F_Q$), whose transfer function includes a linear term ($h_1$), at least one quadratic term ($h_2$) and/or cubic term ($h_3$), and at least one finite time delay ($z^{-1}$),
said Volterra filter i) receiving as an input the EGM signal ($x_i[k]$) of one of said respective EGM channels and ii) generating as an output a corresponding filtered ECG signal ($y_{i,j}[k]$).

**2.** The device of claim 1, comprising:

- a plurality of non-linear Volterra filters (12), each receiving as an input the EGM signal ($x_i[k]$) of one of said respective EGM channels (10), and delivering as an output a corresponding filtered signal ($y_{i,j}[k]$); and
- means (14) for linear combination of the signals filtered by said plurality of non-linear filters, delivering as an output said reconstructed ECG signal ($y_j[k]$).

3. The device of claim 1, comprising:

- for each of the EGM channels: a first linear filter (24) receiving as an input the corresponding EGM signal ($x_1$[$n$], $x_2$[$n$]), a second linear filter (26) receiving as an input the same EGM signal raised to the second power (42), and/or a third linear filter (40) receiving as an input the same signal EGM raised to the third power (44); and
- means (14) for linear combination of the signals filtered by the first, second and/or third linear filters of each of the EGM channels, delivering as an output said reconstructed ECG signal ($y$[$n$]).

4. The device of claim 3, further comprising:

- an additional linear filter (46) receiving as an input a crossed product (48) of the signals of each of the EGM channels;
the linear combination means further combining to the signals filtered by the first, second and/or third linear filters of each of the EGM channels the signal delivered by said additional filter.

5. The device of claim 4, wherein there are two EGM channels, with one atrial EGM channel and one ventricular EGM channel.

6. The device of claim 1, comprising:

- means (50) for concatenating sequences of samples ($x_1$[$n$], $x_2$[$n$] ... $x_Q$[$n$]) produced at a frequency F on each of the Q respective EGM channels, and delivering as an output a concatenated signal ($z$[$n$]) of frequency QF;
- a non-linear Volterra filter (12) receiving as an input said concatenated signal ($z$[$n$]) and delivering as an output a corresponding filtered signal ($w$[$n$]) of frequency QF; and
- means for 1/Q subsampling of said filtered signal ($w$[$n$]), delivering as an output, at a frequency F, said reconstructed ECG signal ($y$[$n$]).

7. The device of claim 1, further comprising means for preliminary determination of the digital filter parameters, comprising:

- means for simultaneously collecting acquired EGM signal (10) and at least one acquired ECG signal (18); and
- means (20, 22) for determining the digital filer parameters, through minimisation of a difference between said acquired ECG signal (18) and an ECG signal (16) reconstructed based on said EGM signals (10) by means of said digital filter (12, 14).

8. The device of claim 7, wherein the digital filter parameter determination means (20, 22) are means for direct calculation of said parameters, adapted to implement a matrix calculation algorithm given a solution verifying a least squares minimisation criterion.

9. The device of claim 8, wherein the digital filter parameter determination means (20, 22) are means adapted to implement a matrix calculation algorithm given a solution verifying a composite criterion that combines the least squares minimisation with a constraint of the Tikhonov regularisation type.

10. The device of claim 7, wherein the digital filter parameter determination means (20, 22) are means for iterative calculation of said parameters, adapted to implement an algorithm of the variable-pitch Recursive Least Squares (RLS) type.

11. The device of claim 1, further comprising means for quality evaluation of the reconstruction operated by the non-linear digital filter, comprising:

- means for simultaneously collecting acquired EGM signals and at least one acquired ECG signal;
- means for determining an ECG signal reconstructed based on said acquired EGM signals; and
- means for calculating a coefficient of correlation between the acquired ECG signal and the reconstructed ECG signal.

12. The device of claim 11, further comprising:

- means for preliminary determination of the digital filter parameters, and

- means for validation of the thus-determined parameters, said means comprising means for comparing to a given threshold the correlation coefficient calculated by said reconstruction quality evaluation means, and for validating or invalidating said parameters based on the result of said comparison.

13. The device of claim 1, wherein said plurality of inputs are inputs adapted to receive a corresponding plurality of EGM signals acquired from electrodes chosen among: distal and/or proximal right ventricular electrode, distal and/or proximal right atrial electrode, distal and/or proximal left ventricular electrode, ventricular or atrial defibrillation coil, supraventricular defibrillation coil.

14. The device of claim 1, wherein said device is an implantable device of the cardiac prosthesis type for stimulation, resynchronisation, cardioversion and/or defibrillation.

15. The device of claim 1, wherein said device is an external programmer comprising means for download and analysis of the EGM signals collected and memorised by an implant.

16. The device of claim 1, wherein said device is a *home monitoring monitor*, comprising means for download and analysis of the EGM signals collected and memorised by an implant, as well as means for automatic remote transmission of the thus-collected data to a remote site.

17. The device of claim 1, wherein said device is a site data server, receiving data from a *home monitoring* remote monitor comprising means for download of the EGM signal collected and memorised by an implant, as well as means for automatic remote transmission of the thus-collected data to said site.


**Patentansprüche**

1. Vorrichtung zur Verarbeitung von Signalen, die für Depolarisations-Herzpotentiale des Herzmuskels repräsentativ sind, die Einrichtungen zur EKG-Rekonstruktion aufweist, mit:

   - einer Vielzahl von Eingängen, die eine entsprechende Vielzahl von EGM-Signalen eines endokavitären oder epikardialen Elektrogramms ($x_1$, $x_2$, ... $x_Q$) empfangen, die je in einem EGM-Kanal (10) erfasst werden;
   - mindestens einem Ausgang, der ein EKG-Signal eines rekonstruierten Oberflächen-Elektrokardiogramms ($y_j$) bezüglich einer gegebenen EKG-Abweichung (16) liefert; und
   - ein nicht-lineares digitales Filter (12, 14; 12', 12", 14; 12, 52) mit einer Transferfunktion, das das EKG-Signal abhängig von der Vielzahl von EGM-Signalen ermitteln kann,

   **dadurch gekennzeichnet, dass** das nicht-lineare digitale Filter ein Filter vom Typ Volterra ($F_1$, $F_2$, ... $F_Q$) enthält, dessen Transferfunktion einen linearen Term ($h_1$), mindestens einen quadratischen ($h_2$) und/oder kubischen Term ($h_3$) und mindestens eine endliche zeitliche Verzögerung ($z^{-1}$) enthält, wobei dieses Volterra-Filter i) am Eingang das EGM-Signal ($x_i[k]$) von einem der EGM-Kanäle empfängt und ii) am Ausgang ein gefiltertes entsprechendes EKG-Signal ($y_{i,j}[k]$) erzeugt.

2. Vorrichtung nach Anspruch 1, die enthält:

   - eine Vielzahl nicht-linearer Volterra-Filter (12), die je am Eingang das EGM-Signal ($x_i[k]$) von einem der EGM-Kanäle (10) empfangen und am Ausgang ein entsprechendes gefiltertes Signal ($y_{i,j}[k]$) liefern; und
   - Einrichtungen (14) zur linearen Kombination der von dieser Vielzahl nicht-linearer Filter gefilterten Signale, die am Ausgang das rekonstruierte EKG-Signal ($y_j[k]$) liefern.

3. Vorrichtung nach Anspruch 1, die enthält:

   - für jeden der EGM-Kanäle: ein erstes lineares Filter (24), das am Eingang das entsprechende EGM-Signal ($x_1[n]$, $x_2[n]$) empfängt, ein zweites lineares Filter (26), das am Eingang das gleiche EGM-Signal ins Quadrat erhoben (42) empfängt, und/oder ein drittes lineares Filter (40), das am Eingang das gleiche EGM-Signal hoch drei genommen (44) empfängt; und
   - Einrichtungen (14) zur linearen Kombination der durch die ersten, zweiten und/oder dritten linearen Filter jedes der EGM-Kanäle gefilterten Signale, die am Ausgang das rekonstruierte EKG-Signal ($y[n]$) liefern.

**4.** Vorrichtung nach Anspruch 3, die außerdem enthält:

- ein zusätzliches lineares Filter (46), das am Eingang ein gekreuztes Produkt (48) der Signale jedes der EGM-Kanäle empfängt;
wobei die Einrichtungen zur linearen Kombination außerdem das von diesem zusätzlichen Filter gelieferte Signal mit den von den ersten, zweiten und/oder dritten linearen Filtern jedes der EGM-Kanäle gefilterten Signalen kombinieren.

**5.** Vorrichtung nach Anspruch 4, wobei es zwei EGM-Kanäle gibt, mit einem aurikulären EGM-Kanal und einem ventrikulären EGM-Kanal.

**6.** Vorrichtung nach Anspruch 1, die enthält:

- Einrichtungen (50), um Tastprobenfolgen ($x_1[n]$, $x_2[n]$, ... $x_Q[n]$) zu verketten, die mit der Frequenz F in jedem der Q EGM-Kanäle erzeugt werden, und am Ausgang ein verkettetes Signal ($z[n]$) der Frequenz QF zu liefern;
- ein nicht-lineares Volterra-Filter (12), das am Eingang das verkettete Signal ($z[n]$) empfängt und am Ausgang ein entsprechendes gefiltertes Signal ($w[n]$) der Frequenz QF liefert; und
- Einrichtungen zur Unterabtastung 1/Q des gefilterten Signals ($w[n]$), die am Ausgang das rekonstruierte EKG-Signal ($y[n]$) auf einer Frequenz F liefern.

**7.** Vorrichtung nach Anspruch 1, die außerdem Einrichtungen zur vorhergehenden Bestimmung der Parameter des digitalen Filters aufweist, die enthalten:

- Einrichtungen zum gleichzeitigen Sammeln der erfassten EGM-Signale (10) und mindestens eines erfassten EKG-Signals (18); und
- Einrichtungen (20, 22) zur Bestimmung der Parameter des digitalen Filters, durch Minimierung eines Abstands zwischen dem erfassten EKG-Signal (18) und einem rekonstruierten EKG-Signal (16) ausgehend von den EGM-Signalen (10) mittels des digitalen Filters (12, 14).

**8.** Vorrichtung nach Anspruch 7, wobei die Einrichtungen (20, 22) zur Bestimmung der Parameter des digitalen Filters Einrichtungen zur direkten Berechnung der Parameter sind, die einen Matrizenrechnungsalgorithmus anwenden können, der eine Lösung ergibt, die ein Kriterium der Minimierung der kleinsten Fehlerquadrate erfüllt.

**9.** Vorrichtung nach Anspruch 8, wobei die Einrichtungen (20, 22) zur Bestimmung der Parameter des digitalen Filters Einrichtungen sind, die einen Matrizenrechnungsalgorithmus anwenden können, der eine Lösung ergibt, die ein Verbundkriterium erfüllt, das die Minimierung der kleinsten Fehlerquadrate mit einer Bedingung von der Art Tikhonov-Regularisierung kombiniert.

**10.** Vorrichtung nach Anspruch 7, wobei die Einrichtungen (20, 22) zur Bestimmung der Parameter des digitalen Filters Einrichtungen zur iterativen Berechnung der Parameter sind, die einen Algorithmus von der Art rekursive kleinste Fehlerquadrate RLS (*Recursive Least Squares*) mit variabler Steigung anwenden können.

**11.** Vorrichtung nach Anspruch 1, die außerdem Einrichtungen zur Bewertung der Qualität der vom nicht-linearen Filter durchgeführten Rekonstruktion aufweist, die enthalten:

- Einrichtungen zum gleichzeitigen Sammeln von erfassten EGM-Signalen und von mindestens einem erfassten EKG-Signal;
- Einrichtungen zur Bestimmung eines rekonstruierten EKG-Signals ausgehend von den erfassten EGM-Signalen; und
- Einrichtungen zur Berechnung eines Korrelationskoeffizienten zwischen dem erfassten EKG-Signal und dem rekonstruierten EKG-Signal.

**12.** Vorrichtung nach Anspruch 11, die außerdem enthält:

- Einrichtungen zur vorhergehenden Bestimmung der Parameter des digitalen Filters, und
- Einrichtungen zur Validierung der so bestimmten Parameter, wobei diese Einrichtungen Einrichtungen enthalten, um den von den Einrichtungen zur Bewertung der Qualität der Rekonstruktion berechneten Korrelationskoeffizienten mit einer gegebenen Schwelle zu vergleichen, und um die Parameter abhängig vom Ergebnis dieses Vergleichs zu validieren oder ungültig zu machen.

13. Vorrichtung nach Anspruch 1, wobei die Vielzahl von Eingängen Eingänge sind, die eine entsprechende Vielzahl von erfassten EGM-Signalen ausgehend von Elektroden empfangen können, die ausgewählt werden unter: distale und/oder proximale rechte ventrikuläre Elektrode, distale und/oder proximale rechte aurikuläre Elektrode, distale und/oder proximale linke ventrikuläre Elektrode, ventrikuläre oder aurikuläre Defibrillationsspule; supraventrikuläre Defibrillationsspule.

14. Vorrichtung nach Anspruch 1, wobei die Vorrichtung eine implantierbare Vorrichtung von der Art Herzprothese zur Stimulation, Resynchronisation, Kardioversion und/oder Defibrillation ist.

15. Vorrichtung nach Anspruch 1, wobei die Vorrichtung ein externes Programmiergerät ist, das Einrichtungen zuum Herunterladen und zur Analyse der von einem Implantat gesammelten und gespeicherten EGM-Signale ist.

16. Vorrichtung nach Anspruch 1, wobei die Vorrichtung ein *Home-Monitoring-Monitor* ist, der Einrichtungen zum Herunterladen der von einem Implantat gesammelten und gespeicherten EGM-Signale sowie Einrichtungen zur automatischen Fernübertragung der so gesammelten Daten an einen fernen Standort enthält.

17. Vorrichtung nach Anspruch 1, wobei die Vorrichtung ein Datenserver eines Standorts ist, der Daten von einem fernen *Home-Monitoring*-Monitor empfängt, der Einrichtungen zum Herunterladen der von einem Implantat gesammelten und gespeicherten EGM-Signale sowie Einrichtungen zur automatischen Fernübertragung an den Standort der so gesammelten Daten enthält.

FIG_1

FIG_2

FIG_3

## FIG_4

## FIG_5

## RÉFÉRENCES CITÉES DANS LA DESCRIPTION

**Documents brevets cités dans la description**

- US 5740811 A1, Hedberg
- US 6980850 A1, Kroll
- US 7383080 B

- US 20080065161 A
- EP 1902750 A1
- WO 2008008692 A2

**Littérature non-brevet citée dans la description**

- **SCHETZEN M.** The Volterra and Wiener Theories of Nonlinear Systems. Wiley and Sons, 1980
- **MATHEWS VJ.** *Adaptive Polynomial Filters, IEEE Signal Processing Magazine,* Juillet 1991, vol. 8 (3), 1 0-26

- Recursive Least Squares. **HAYES, MH.** Statistical Digital Signal Processing and Modeling. Wiley, 1996, 541
- **HAYKIN S.** Adaptive Filter Theory. Prentice Hall, 2002